# EUROPEAN PATENT APPLICATION

(11) **EP 4 279 495 A1**
(43) Date of publication of application: **22.11.2023**
(21) Application number: 22174524.3
(22) Date of filing: 20.05.2022
(51) Int. Cl.: C07H 1/00, C07H 15/04

(54) **PROCESS OF MANUFACTURING ALKYLPOLYGLYCOSIDE**

(71) Applicant: BASF SE, 67056 Ludwigshafen am Rhein (DE)
(72) Inventor: BUSCH, Stefan, 40589 Düsseldorf (DE); MAHNKE, Eike Ulf, 40589 Düsseldorf (DE); BENERT, Arnold, 40589 Düsseldorf (DE)
(74) Representative: BASF IP Association

(57) **Abstract**

Presently claimed invention is directed to a process for the preparation of an alkylpolyglycoside in the presence of a sulfonated fatty acid as catalyst.

## Description

### Field of invention

Presently claimed invention is directed to a process for the preparation of an alkylpolyglycoside and product obtained thereof.

### Background of the invention

Alkylpolyglycosides (APGs) command great interest in many of the industry because of their low toxicity, their very good biological degradability, and their interesting practical properties. According to F. A. Hughes and B. W. Lew, J.A.O.C.S. 47 (1970), 162, the properties of the alkyloligoglycosides are strongly determined by their degree of oligomerization. For this reason, there is interest in processes by which alkyl oligoglycosides with definite average degrees of oligomerization can be selectively prepared. The current processes for the preparation of alkylpolyglycosides are partly based on renewable raw materials. The terms alkylpolyglycosides and alkyl oligo glycosides are used as synonyms.

Aldoses (reducing sugar) can be converted smoothly to an alkylpolyglycoside by the Fischer glycoside synthesis with hydrophilic alcohols such as methanol or ethanol and acid catalysis. With hydrophobic alcohols, on the other hand, solubility problems occur.

The acetalisation reaction needs to be catalysed by strong acids to proceed with acceptable speed.

According to U.S. Pat. No. 3,219,656, higher alkylglucosides are obtained by first preparing butylglucoside, which is then subjected to alcoholysis with a higher alcohol. This process requires large amounts of cation exchanger. XE-230 is used as a catalyst, which is a sulfonated styrene-divinylbenzene copolymer which has high divinylbenzene content acting as a crosslinker of the resin.

German OS No. 19 43 689 discloses that higher alkyl oligosaccharides can be prepared from butylglycoside by transacetalization with alcohols containing 11 to 32 carbon atoms and acid catalysis, the preferred catalyst is sulfuric acid. The degree of oligomerization is inversely proportional to the moles of alcohol used per mole of butylglucoside. available.

US 4,866,165 A also discloses a process for the preparation of alkyl oligoglycosides with sulfuric acid as catalyst. Usually, at completion of the APG synthesis the used catalysts (acids) are deactivated by neutralisation, the resulting salt(s) remain in the final APG product.

Many well-proven catalysts (LABSA (linear alkyl benzene sulfonic acid), pTSA (para-toluene sulfonic acid)) are of petro-based origin and therefore not positively evaluated according to current Ecolabels. Sulfosuccinic acid could be an alternative but ruled out because it is of GMO-origin. Sulfuric acid, a mineral alternative and the main catalyst of the cited prior art, is converted to alkyl sulfate upon deactivation which is undesirable as it is an unwanted impurity in APGs especially for cosmetic applications and especially as APGs are offered as surfactants with neglectable amounts of sulfate.

Thus, first object of the presently claimed invention is to provide a process for the preparation of alkylpolyglycosides which can either reduce or eliminate the formation of alkylsulfate as impurity in the final alkylpolyglycoside.

Another object of the presently claimed invention is to provide a process which can either reduce or eliminate the formation of polysaccharides (e.g. polydextrose in case of glucose) as by-product.

Yet, another object of the presently claimed invention is to provide a process where the entire reactants are derived from renewable resources, thus the product is in compliance to Ecolabel standards. The new process should allow to substitute the currently used catalyst without changing the specification of the final product.

### Summary of invention

Surprisingly, alpha-sulfo fatty acid (SFA) was found to catalyse APG-formation efficiently without substantially reducing reaction time or forming excessive amounts of polysaccharides (e.g. polydextrose in case of glucose). When neutralised at the end of the reaction, SFA is converted into its salt which is an acceptable component in APGs as it is a sulfonate salt (not a sulfate) and derived from renewable raw materials since it is prepared by sulfonation of naturally sourced fatty acids, thus only contains 'natural' carbon atoms. In addition, the presence of the SFA salt, which remains in the finished product results in an APG-product with the same surfactant properties as the APG manufactured with the previous catalyst (LABSA).

It was found that using a process for the preparation of an alkylpolyglycoside comprising at least the steps of reacting at least one saccharide (S) with at least one alcohol (A) in the presence of at least one acid of formula (I)

R¹-CH(SO₃H)-COOG formula (I)

wherein G is selected from hydrogen, or substituted or unsubstituted, linear or branched C₁-C₃₀ alkyl or substituted or unsubstituted, linear or branched C2-C30 alkenyl and R¹ is a substituted or unsubstituted, linear or branched C₁-C₃₀ alkyl can lead to achieve above objectives. Unlike sulfuric acid the SFA concentration in the slurry seems not to affect polysaccharide formation greatly, thus allowing for fine-tuning the APGs' surfactant performance by adjusting the co-emulsifier introduced into the APG by use of varying amounts of SFA.

Thus, in a first aspect, the presently claimed invention is directed to a process for the preparation of an alkylpolyglycoside comprising at least the steps of reacting at least one saccharide (S) with at least one alcohol (A) in the presence of at least one acid of formula (I)

R¹-CH(SO₃H)-COOG formula (I)

wherein G is selected from hydrogen, or substituted or unsubstituted, linear or branched C₁-C₃₀ alkyl or substituted or unsubstituted, linear or branched C2-C30 alkenyl, and R¹ is substituted or unsubstituted, linear or branched, C₁-C₃₀ alkyl.

The second aspect of the presently claimed invention is directed to an alkylpolyglycoside obtained according to the first aspect.

The third aspect of the presently claimed invention is directed to a composition comprising the alkylpolyglycoside according to second aspect.

### Detailed Description

Before the present compositions and formulations of the presently claimed invention are described, it is to be understood that this invention is not limited to particular compositions and formulations described, since such compositions and formulation may, of course, vary. It is also to be understood that the terminology used herein is not intended to be limiting, since the scope of the presently claimed invention will be limited only by the appended claims.

If hereinafter a group is defined to comprise at least a certain number of embodiments, this is meant to also encompass a group which preferably consists of these embodiments only. Furthermore, the terms 'first', 'second', 'third' or 'a', 'b', 'c', etc. and the like in the description and in the claims, are used for distinguishing between similar elements and not necessarily for describing a sequential or chronological order. It is to be understood that the terms so used are interchangeable under appropriate circumstances and that the embodiments of the presently claimed invention described herein are capable of operation in other sequences than described or illustrated herein. In case the terms 'first', 'second', 'third' or '(A)', '(B)' and '(C)' or '(a)', '(b)', '(c)', '(d)', 'i', 'ii' etc. relate to steps of a method or use or assay there is no time or time interval coherence between the steps, that is, the steps may be carried out simultaneously or there may be time intervals of seconds, minutes, hours, days, weeks, months or even years between such steps, unless otherwise indicated in the application as set forth herein above or below.

Furthermore, the ranges defined throughout the specification include the end values as well i.e. a range of 1 to 10 implies that both 1 and 10 are included in the range. For the avoidance of doubt, applicant shall be entitled to any equivalents according to applicable law.

In the following passages, different aspects of the presently claimed invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to 'one embodiment' or 'an embodiment' means that a particular feature, structure or characteristic described in connection with the embodiment is included in at least one embodiment of the presently claimed invention. Thus, appearances of the phrases 'in one embodiment' or 'in an embodiment' in various places throughout this specification are not necessarily all referring to the same embodiment, but may.

Furthermore, the particular features, structures or characteristics may be combined in any suitable manner, as would be apparent to a person skilled in the art from this disclosure, in one or more embodiments. Furthermore, while some embodiments described herein include some, but not other features included in other embodiments, combinations of features of different embodiments are meant to be within the scope of the presently claimed invention, and form different embodiments, as would be understood by those in the art. For example, in the appended claims, any of the claimed embodiments can be used in any combination.

In a first embodiment, the presently claimed invention is directed to a process for the preparation of an alkylpolyglycoside comprising at least the steps of reacting at least one saccharide (S) with at least one alcohol (A) in the presence of at least one acid of formula (I)

R¹-CH(SO₃H)-COOG formula (I)

wherein G is selected from hydrogen, or substituted or unsubstituted, linear or branched C₁-C₃₀ alkyl or substituted or unsubstituted, linear or branched C₂-C₃₀ alkenyl; preferably G is selected from hydrogen, or linear or branched C₂ to C₂₄ alkyl, or the alkyl moiety of (A),
more preferably G is selected from hydrogen, or C₆ to C₂₂ or the alkyl moiety of (A) and specifically G is selected from linear C₈/C₁₀-alkyl or linear C₁₂/C₁₄-alkyl or linear C₁₆/C₁₈-alkyl, mostly preferred G is hydrogen,
   and
R¹ is substituted or unsubstituted, linear or branched, C₁-C₃₀ alkyl.

Preferably R¹ is a linear or branched C₄ to C₂₂-alkyl, more preferably the fraction in which the radical R¹ is an alkenyl moiety based on the mass of the R¹ moiety - is 3% by weight or less. Even more preferably R¹ is a saturated, linear C₁₀ to C₁₆-alkyl, specifically R¹ is a saturated, linear C₁₀ to C₁₆-alkyl with 90% by weight or more of a decyl and/or a dodecyl moiety - based on the total mass of the moiety R¹,
more specifically R¹ is a saturated, linear C₁₀ to C₁₆-alkyl with 65 to 75% by weight decyl and 20 to 30% by weight dodecyl moiety - based on the total mass of the moiety R¹.

Preferably, the presently claimed invention is directed to a process for the preparation of an alkylpolyglycoside comprising at least the steps of reacting at least one saccharide (S) with at least one alcohol (A) in the presence of at least one acid of formula (I)

R¹-CH(SO₃H)-COOG formula (I)

wherein G is selected from hydrogen, or substituted or unsubstituted, linear or branched C₂-C₂₄ alkyl or substituted or unsubstituted, linear or branched C₂-C₃₀ alkenyl; and
R¹ is substituted or unsubstituted, linear or branched, C₁₀-C₁₆ alkyl.

More preferably, the presently claimed invention is directed to a process for the preparation of an alkylpolyglycoside comprising at least the steps of reacting at least one saccharide (S) with at least one alcohol (A) in the presence of at least one acid of formula (I)

R¹-CH(SO₃H)-COOG formula (I)

wherein G is selected from hydrogen, or the alkyl moiety of (A) and
R¹ is substituted or unsubstituted, linear or branched, C₁₀-C₁₆ alkyl.

Most preferably, the presently claimed invention is directed to a process for the preparation of an alkylpolyglycoside comprising at least the steps of reacting at least one saccharide (S) with at least one alcohol (A) in the presence of at least one acid of formula (I) moiety

R¹-CH(SO₃H)-COOG formula (I)

wherein G is selected from hydrogen, or linear C₈/C₁₀-alkyl or linear C₁₂/C₁₄-alkyl or linear C₁₆/C₁₈-alkyl; and
R¹ is a saturated, linear C₁₀ to C₁₆-alkyl with 90% by weight or more of a decyl and/or a dodecyl moiety - based on the total mass of the moiety R¹.

In particular, the presently claimed invention is directed to a process for the preparation of an alkylpolyglycoside comprising at least the steps of reacting at least one saccharide (S) with at least one alcohol (A) in the presence of at least one acid of formula (I)

R¹-CH(SO₃H)-COOG formula (I)

wherein G is hydrogen; and
R¹ is a saturated, linear C₁₀ to C₁₆-alkyl with 65 to 75% by weight decyl and 20 to 30% by weight dodecyl moiety - based on the total mass of the moiety R¹.

In one embodiment, the unsubstituted linear alkyl moiety of R¹ is preferably selected from, but are not limited to, methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, docosanyl, cocoyl, cetearyl, palmitoleyl, oleyl, erucyl more preferably selected from the group consisting of hexyl, octyl, decyl, dodecyl, tetradecyl, hexadecyl, heptadecyl, octadecyl, or eicosyl.

In one embodiment, the unsubstituted linear alkyl moiety of G and/or A is preferably selected from, but are not limited to, methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, docosanyl, cocyl, cetearyl, palmitoleyl, oleyl, erucyl more preferably selected from the group consisting of hexyl, octyl, decyl, dodecyl, tetradecyl, hexadecyl, heptadecyl, octadecyl, or eicosyl.

In another embodiment, the unsubstituted branched alkyl moiety of G and/or A is selected from, but is not limited to, isopropyl, iso-butyl, iso-amyl, neo-pentyl, iso-hexyl, iso-heptyl, iso-octyl, iso-nonyl, iso-decyl, iso-dodecyl, iso-tridecyl, iso-tetradecyl, iso-hexadecyl, iso-octadecyl, isostearyl, iso-eicosyl, 2-butyl-octyl, 2-pentyl-nonyl, 2-ethyl-hexyl, 2-propyl-heptyl, 2-Isopropyl-5-methylhexyl (iso-amyl guerbetoyl), 2-butyl-octyl, 2-pentyl-nonyl, 2-hexyl-decyl, 2-octyl-dodecyl; more preferably selected from the group consisting of 2-ethyl-hexyl, 2-propyl-heptyl, 2-hexyl-decyl, 2-octyl-dodecyl and mixtures thereof.

In one preferred embodiment the saccharide (S) is selected from monosaccharides, oligosaccharides, polysaccharides, or a mixture thereof, more preferably the saccharide (S) is selected from monosaccharides, oligosaccharides, or a mixture thereof, and most preferably the saccharide (S) is monosaccharides.

In another preferred embodiment the monosaccharides are selected from galactose, glucose, glyceraldehyde, fructose, ribose, xylose, or a mixture thereof, more preferably the monosaccharides are selected from galactose, glucose, fructose, ribose, xylose or a mixture thereof, even more preferably the monosaccharides are selected from glucose, fructose, xylose or a mixture thereof, most preferably the monosaccharides are selected from glucose, xylose or a mixture thereof, and in particular the monosaccharide is glucose.

The alcohol (A) is selected from a primary alcohol, secondary alcohol, tertiary alcohol, or a combination of two or more, preferably the alcohol (A) is selected from primary alcohol, secondary alcohol, or a combination thereof, and most preferably the alcohol (A) is primary alcohol.

Preferably the alcohol is a fatty alcohol, a guerbet alcohol, an alkoxylated, preferably ethoxylated or propoxylated alcohol, most preferably the alcohol (A) is a fatty alcohol, or a mixture of two or more of the aforementioned.

The alcohol (A) is a compound of formula (II)

R²-OH formula (II)

wherein R² is selected from substituted or unsubstituted, linear or branched C₁-C₃₀ alkyl, or substituted or unsubstituted, linear or branched C₃-C₃₀ alkenyl or an C₂ -C₆ alkyl substituted with one or more hydroxyl groups, specifically R²-OH is glycerine.

More preferably the alcohol (A) is a compound of formula (II)

R²-OH formula (II)

wherein R² is selected from a linear or branched C₂ to C₂₄ alkyl or unsubstituted, linear or branched C₃-C₃₀ alkenyl.

Most preferably the alcohol (A) is a compound of formula (II)

R²-OH formula (II)

wherein R² is selected from C₆ to C₂₂ or unsubstituted, linear or branched C₁₂-C₂₀ alkenyl.

In particular the alcohol (A) is a compound of formula (II)

R²-OH formula (II)

wherein R² is selected from linear C₈/C₁₀-alkyl or linear C₁₂/C₁₄-alkyl or linear C₁₆/C₁₈-alkyl or linear C₁₈-alkenyl.

More preferably the alcohol (A) is a compound of formula (II) is selected from methanol, ethanol, propanol, isopropanol, butanol, isoamyl alcohol, hexanol, octanol, decanol, dodecanol, isotridecanol, tetradecanol, hexadecanol, octadecanol, icosanol, docosanol, cocoyl alcohol, cetearyl alcohol, isostearyl alcohol, palmitoleyl alcohol, oleyl alcohol, erucyl alcohol, glycerol, trimethylolpropane, pentaerythritol, glycol, propylenglycol, butyleneglycol, 2-ethyl-hexanol, 2-propyl-heptanol, 2-isopropyl-5-methylhexanol (isoamyl guerbet alcohol), 2-butyl-octanol, 2-pentyl-nonanol, 2-hexyl-decanol, 2-octyl-dodecanol or a mixture of two or more thereof.

In another preferred embodiment, a compound of formula (I) is prepared from suitable carboxylic acid or its ester as precursor comprising at least a step of sulfonation reaction. The precursor carboxylic acid preferably selected from

R¹-CH₂-COOH formula (IV)

The compound of formula (I) can be prepared by all methods known appropriately to the person skilled in the art. A particularly preferred method of preparation is the sulfonation of the corresponding carboxylic acids according to formula IV. Here, the corresponding carboxylic acid and in particular the corresponding fatty acids are reacted with gaseous sulfur trioxide, the sulfur trioxide being used preferably in an amount such that the molar ratio of SO₃ to fatty acid is in the range from 1: 1 to 1.1: 1.

In another preferred embodiment, the process for the preparation of an alkylpolyglycoside is carried out at a temperature in the range of 40 °C to boiling point of alcohol (A) at the pressure the reaction is carried out.

In another preferred embodiment, the process for the preparation of an alkylpolyglycoside is carried out at a temperature in the range of 40 °C to 200 °C, more preferably the process for the preparation of an alkylpolyglycoside is carried out at a temperature in the range of 80 °C to 140 °C, most preferably the process for the preparation of an alkylpolyglycoside is carried out at a temperature in the range of 95 °C to 130 °C, and in particular the process for the preparation of an alkylpolyglycoside is carried out at a temperature in the range of 100 °C to 120 °C.

In another preferred embodiment, the process for the preparation of an alkylpolyglycoside is carried out at a pressure in the range of 0.1 mbar to 900 mbar, more preferably the process for the preparation of an alkylpolyglycoside is carried out at a pressure in the range of 5 mbar to 500 mbar, and more preferably the process for the preparation of an alkylpolyglycoside is carried out at a pressure in the range of 10 mbar to 100 mbar.

In another preferred embodiment, the process for the preparation of an alkylpolyglycoside is carried out at in presence of at least one solvent or in the absence of a solvent, more preferably in the absence of a solvent.

In another preferred embodiment, the process for the preparation of an alkylpolyglycoside, the pH of the reaction mixture is maintained at ≤3.5, more preferably the pH of the reaction mixture is maintained at ≤ 3.0,

In another preferred embodiment, the reaction mixture comprises the acid of formula (I) in an amount of at least 2 micromole based on per gram weight of the mixture of the saccharide (S) and the alcohol(A), More preferably the reaction mixture comprises the acid of formula (I) in the range of 2 to 100 micromole based on per gram weight of the mixture of the saccharide (S) and the alcohol(A), even more preferably the reaction mixture comprises the acid of formula (I) in the range of 2 to 50 micromole based on per gram weight of the mixture of the saccharide (S) and the alcohol(A), and most preferably the reaction mixture comprises the acid of formula (I) in the range of 3 to 20 micromole based on per gram weight of the mixture of the saccharide (S) and the alcohol(A),

In another preferred embodiment, the mol equivalent ratio of alcohol(A) to saccharide (S) is in the range of 1.0 to 10, more preferably from 1.5 to 7 and most preferably from 2 to 5.

Usually, the process according to any one of the embodiments further comprises a step of isolating the alkylpolyglycoside from the reaction mixture and optionally it further comprises a step of purifying the alkylpolyglycoside.

Hence in another preferred embodiment, the alkylpolyglycoside formed in the reaction is isolated and purified by any method known in the art selected from chemical separation, acid-base neutralization, distillation, evaporation, column chromatography, filtration, concentration, crystallization, re-crystallization, or a combination thereof. A person skilled in the art is aware of such techniques.

In another embodiment, the presently claimed invention is directed an alkylpolyglycoside obtained according the process described above.

In another embodiment, the presently claimed invention is directed an alkylpolyglycoside obtained according the process described above that is diluted with water.

In another preferred embodiment, the alkylpolyglycoside obtained according to the process described above has a degree of polymerization greater than 1. More preferably the alkylpolyglycoside has an average degree of polymerization in the range of 1.1 to 5, even more preferably the alkylpolyglycoside has an average degree of polymerization in the range of 1.1 to 4, most preferably the alkylpolyglycoside has an average degree of polymerization in the range of 1.1 to 3, and in particular the alkylpolyglycoside has an average degree of polymerization in the range of 1.1 to 2.

In another preferred embodiment, the alkylpolyglycoside obtained according to the process described above comprises at least one sulfonate impurity of the compound of formula (I) in free form or a salt form is present in an amount of ≤ 5.0 wt.% based on overall weight of the dry residue of the distilled alkylpolyglycoside. More preferably the alkylpolyglycoside comprises at least one sulfonate impurity of the compound of formula (I) in free form or a salt form is present in an amount of 0.01 to ≤ 3.0 wt.% based on overall weight of the alkylpolyglycoside, even more preferably the alkylpolyglycoside comprises at least one sulfonate impurity of the compound of formula (I) in free form or a salt form is present in an amount of ≥0.05 to ≤ 2.0 wt.% based on overall weight of the alkylpolyglycoside, most preferably the alkylpolyglycoside comprises at least one sulfonate impurity of the compound of formula (I) in free form or a salt form is present in an amount of ≥ 0.1 to ≤ 1.0 wt.% based on overall weight of the alkylpolyglycoside, particular preferably the alkylpolyglycoside comprises at least one sulfonate impurity of the compound of formula (I) in free form or a salt form is present in an amount of ≥ 2.0 to ≤ 4.0 wt.% based on overall weight of the alkylpolyglycoside.

In another preferred embodiment, the alkylpolyglycoside obtained according to the process described above comprises a polysaccharide by-product (formed by polymerization of the used carbohydrate) in an amount of ≤15.0 wt.% based on overall weight of the alkylpolyglycoside, more preferably the alkylpolyglycoside comprises a polydextrose impurity is present in an amount in the range of ≥0.01 to ≤10.0 wt.% based on overall weight of the alkylpolyglycoside, even more preferably the alkylpolyglycoside comprises a polydextrose impurity is present in an amount in the range of ≥1.0 to ≤7.0 wt.% based on overall weight of the alkylpolyglycoside, preferably the alkylpolyglycoside comprises a polydextrose impurity is present in an amount in the range of ≥2.0 to ≤ 4.0 wt.% based on overall weight of the alkylpolyglycoside, based on overall weight of the alkylpolyglycoside.

The presently claimed invention offers one or more of following advantages:
The novel synthesis route, as described hereinabove, has several advantages over the current state of the art. The process uses all the reactants and catalyst based on renewable resources and reduces the carbon footprint of the process and the product. The alkylpolyglycoside has the same low polydextrose impurity in the final product.

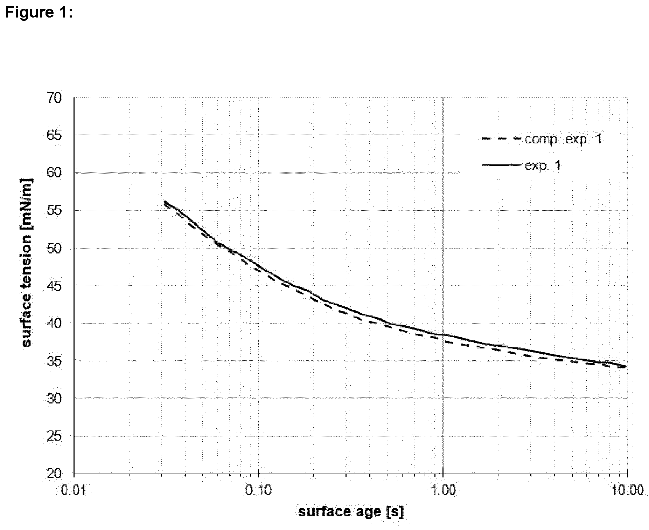

As the catalyst remains in the finished product it has a more or less strong influence on the finished product properties. In case of alkylpolyglycosides the main effect is expressed in its surfactant properties, so that it was necessary to find a substitute for the petrobased catalysts - here linear alkyl benzene sulfonic acid (LABSA) - which does alter the main properties of the finished product to the same extent. Surprisingly this could be achieved by using the catalyst according to formula (I). This exchange of catalyst resulted in an APG product with similar surfactant properties as seen in figure 1 providing the comparison of the dynamic surface tension of aqueous solutions of two alkylpolyglycosides (0.1 wt% of dry residue) - obtained from standard (LABSA) process and according to the invention - at pH 4.9 at room temperature (23 ± 1 °C).

With this catalyst it is not necessary to purify the finished product. After neutralization, distillation and bleaching the finished product comprises the catalyst in salt form. It could be proven that the dynamic surface tension (see figure 1 - dynamic surface tension) of the finished product was not altered by the exchange of the catalyst despite the aromatic structure of the petro-based catalyst.

### Examples

The presently claimed invention is further illustrated in combination with the following examples. These examples are provided to exemplify the presently claimed invention, but are not intended to restrict the scope of the presently claimed invention in any way. The below listed chemicals are used and all are commercially available.

### Materials

- APG: alkylpolyglucoside or alkylpolyglycoside
- DMH: dextrose monohydrate
- DP: degree of polymerisation
- LABSA: linear alkyl benzene sulfonic acid
- FA: fatty alcohol
- HEW: hydroxy equivalent weight
- SFA: α-sulfo fatty acid (acidic form)
- SFA salt: salt of SFA

### Raw materials

### Carbohydrates:

### Dextrose monohydrate (Dextrose Monohydrat M) was obtained from Roquette

### Alcohols:

Fatty alcohol mixtures (C_{8/10} and C_{12/14}) were taken from BASF as explained in experimental data. For sake of comparability of the experiments the compositions of the alcohol mixture were analysed by GC and from this a hydroxy equivalent weight (HEW) was determined for each mixture.

### Catalysts:

□ LABSA was obtained as Maranil DBS/LC (BASF)
□ SFA
   The synthesis of SFA was carried out as described in US5329030 (example 3, column 5) 'sulfonation of tallow fatty acid' at a sulfonation temperature of 75 °C instead of 90 °C. A native fatty acid (C_{12/14} = 70:30 wt%) was used instead of tallow acid and the neutralisation step omitted. The obtained SFA was degassed to remove any dissolved SO₂ /SO₃ by being stored for 2 days at 60 °C.

### Test methods

- pH: standard commercial pH meter from a solution of 10% of the test sample in 90% IPA/water (15:85).
- Distribution of glycosides (for calculation of the DP) and polydex content were carried out by HPLC as described in EP 2 998 311 A1, paragraph [0013].
- Dynamic surface tension was measured by use of a bubble tensiometer SITA online t60 (SITA Messtechnik GmbH)

### Example 1:

1214 g of C_{8/10} fatty alcohol (59.4% C₈, 39.4% C₁₀; 8.49 mol alcohol) and 721 g (3.64 mol) of dextrose monohydrate (carbohydrate) were charged to a 4 I-vessel equipped with stirrer, condensor and phase separator. The resulting slurry was dried under stirring at 75 °C at a pressure of 30 mbar for 30 minutes before a solution of 3.23 g (0.012 mol) SFA in 63 g (0.44 mol) reactant alcohol (catalyst solution) was added through a dropping funnel over 30 minutes. The reaction mixture was heated under stirring to 103 °C (reaction temperature) at 30 mbar separating the reaction water from the mixture whilst returning distilled alcohol back into the reactor. The acetalisation is finished when no more water (approximately the theoretical amount) was collected. The catalyst was deactivated by the addition of 0.65 g MgO and 1.8 g sodium hydroxide at 80 °C under nitrogen. The pH of the APG/excess alcohol was 10.2. The excess alcohol by distillation. The product had a DP of 1.55 and a polydextrose content of 2.1%.

### Example 2:

Example 2 was carried out same as example 1 using 1484 g of C_{12/14} fatty alcohol (65.2% C₁₂, 27.4% C₁₄, 1.0% C₁₆; 7.15 mol alcohol) and 450 g (2.27 mol) dextrosemonohydrate. The reaction was run at 106 °C after addition of 3.34 g (0.012 mol) SFA in 63 g (0.29 mol) of alcohol. The mixture was neutralised with 0.48 g MgO and 1.8 g NaOH (→ pH= 8.8) and distilled to yield an APG with an average DP of 1.48 and a polydextrose content of 4.7%.

### Comparative experiment 1 - with

### C_{8/10} FA and DMH (NPEM00072-ABE-0041)

1167 g of C_{8/10} fatty alcohol (59.4% C₈, 39.4% C₁₀, 8.16 mol alcohol) and 720 g (3.63 mol) of dextrose monohydrate (carbohydrate) are charged to a 4 I-vessel equipped with stirrer, condenser and phase separator. The resulting slurry is dried under stirring at 75 °C at a pressure of 30 mbar for 30 minutes be-fore a solution of 5.4 g LABSA in 108 g (0.76 mol) reactant alcohol (catalyst solution) is added through a dropping funnel over 30 minutes. The reaction mixture is then heated under stirring to 103 °C (reaction temperature) at 30 mbar separating the reaction water from the mixture whilst returning distilled alcohol back into the reactor. The acetalisation is finished when no more water (approximately the theoretical amount) is collected. Addition of 0.65 g MgO and 2.8 g sodium hydroxide at 80 °C under nitrogen deactivates the catalyst. The pH of the APG/excess alcohol mixture was 9.6. After removal of the excess alcohol by distillation a DP of 1.59 and a polydextrose content of 2.8% were found.

## Claims

1. A process for the preparation of an alkylpolyglycoside comprising at least the steps of reacting at least one saccharide (S) with at least one alcohol (A) in the presence of at least one acid of formula (I)
R¹-CH(SO₃H)-COOG formula (I)
wherein G is selected from hydrogen, or substituted or unsubstituted, linear or branched C₁-C₃₀ alkyl or substituted or unsubstituted, linear or branched C₂-C₃₀ alkenyl and R¹ is a substituted or unsubstituted, linear or branched C₁-C₃₀ alkyl.

2. The process according to claim 1, wherein the saccharide (S) is selected from monosaccharides, oligosaccharides, polysaccharides, or a mixture thereof.

3. The process according to claim 2, wherein the monosaccharides are selected from galactose, glucose, glyceraldehyde, fructose, ribose, xylose, or a mixture thereof.

4. The process according to any one of claims 1 to 3, wherein the alcohol (A) is selected from a primary alcohol or a secondary alcohol.

5. The process according to claim 4, wherein the alcohol (A) is a compound of formula (II)
R²-OH formula (II)
wherein R² is selected from substituted or unsubstituted, linear or branched C₁-C₃₀ alkyl, or substituted or unsubstituted, linear or branched C₃-C₃₀ alkenyl or an or an C₂-C₆ alkyl substituted alkyl substituted with one or more hydroxyl groups, specifically R²-OH is glycerine

6. The process according to any one of the claims 1 to 5, comprising at least the steps of reacting at least one saccharide (S) with at least one alcohol (A) in the presence of at least one acid of formula (I)
R¹-CH(SO₃H)-COOG formula (I)
wherein G is selected from hydrogen, or substituted or unsubstituted, linear or branched C₂-C₂₄ alkyl or substituted or unsubstituted, linear or branched C₂-C₃₀ alkenyl; and
R¹ is substituted or unsubstituted, linear or branched, C₁₀-C₁₆ alkyl.

7. The process according to any one of the claims 1 to 6, comprising at least the steps of reacting at least one saccharide (S) with at least one alcohol (A) in the presence of at least one acid of formula (I)
R¹-CH(SO₃H)-COOG formula (I)
wherein G is selected from hydrogen, or the alkyl moiety of (A) and
R¹ is substituted or unsubstituted, linear or branched, C₁₀-C₁₆ alkyl.

8. The process according to any one of the claims 1 to 7, wherein the reaction is carried out at a temperature in the range of 40 °C to boiling point of alcohol (A) at the pressure the reaction is carried out.

9. The process according to any one of the claims 1 to 8, wherein the reaction mixture is maintained at a pH ≤ 3.5.

10. The process according to any one of the claims 1 to 9, wherein the reaction mixture comprises the acid of formula (I) in an amount of at least 2 micromoles based on per gram weight of the mixture of the saccharide (S) and the alcohol (A).

11. The process according to any one of the claims 1 to 10, wherein the mol equivalent ratio of alcohol (A) to saccharide (S) is in the range of 1.0 to 10.

12. An alkylpolyglycoside obtained according any one of the claims 1 to 11.

13. The alkylpolyglycoside according to claim 12, wherein the alkylpolyglycoside has a degree of polymerization greater than 1.

14. The alkylpolyglycoside according to any of claims 12 or 13, wherein the alkylpolyglycoside comprises at least one sulfonate impurity of the compound of formula (I) in an amount of ≤ 5.0 wt.% based on overall weight of the alkylpolyglycoside.

15. The alkylpolyglycoside according to any one of the claims 12 to 14, wherein the alkylpolyglycoside comprises a polysaccharide by-product in an amount of ≤ 15.0 wt.% based on overall weight of the alkylpolyglycoside.
